# EUROPÄISCHE PATENTSCHRIFT

(11) **EP 2 850 547 B1**
(45) Veröffentlichungstag und Bekanntmachung des Hinweises auf die Patenterteilung: **30.09.2020**
(21) Anmeldenummer: 13723413.4
(22) Anmeldetag: 14.05.2013
(51) Int. Cl.: G16H 40/63

(54) **VORRICHTUNG UND VERFAHREN ZUR ÜBERNAHME EINES VORGESCHLAGENEN STANDARDWERTS IN DIE STEUERVORRICHTUNG EINES MEDIZINISCHEN GERÄTS**
DEVICE AND METHOD FOR ACCEPTING A PROPOSED STANDARD VALUE IN THE CONTROL DEVICE OF A MEDICAL APPARATUS
DISPOSITIF ET MÉTHODE POUR L'ADOPTION D'UNE VALEUR STANDARD PROPOSÉE DANS LE DISPOSITIF DE CONTRÔLE D'UN APPAREIL MÉDICAL

(30) Priorität: 14.05.2012 DE 102012009517; 14.05.2012 US 201261646512 P
(43) Veröffentlichungstag der Anmeldung: 25.03.2015
(73) Patentinhaber: Fresenius Medical Care Deutschland GmbH, 61352 Bad Homburg (DE)
(72) Erfinder: WEHMEYER, Wolfgang, 72076 Tübingen (DE)
(74) Vertreter: Dreyhsig, Jörg
(86) Internationale Anmeldenummer: PCT/EP2013/001407
(87) Internationale Veröffentlichungsnummer: WO 2013/170948

(56) Entgegenhaltungen:
- EP-A1- 0 862 159
- WO-A1-2006/131775
- US-A1- 2002 173 721
- US-A1- 2008 294 458
- US-B1- 7 499 862

## Beschreibung

### Technisches Gebiet

Die Erfindung betrifft das Gebiet Vorrichtung und Verfahren zur Parametrisierung von Steuervorrichtungen medizinischer Geräte.

### Stand der Technik

Unter medizinischen Geräten werden insbesondere auch Fluidbehandlungsgeräte wie Blutbehandlungsgeräte verstanden, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte. Ein solches Blutbehandlungsgerät ist Gegenstand der DE 198 49 787 C1 der Anmelderin.

Die Dialyse ist ein Verfahren zur Blutreinigung von Patienten mit akuter oder chronischer Niereninsuffizienz. Grundsätzlich unterscheidet man hierbei Verfahren mit einem extrakorporalen Blutkreislauf, wie die Hämodialyse, die Hämofiltration oder die Hämodiafiltration (im Folgenden unter dem Begriff Hämodialyse zusammengefasst) und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Dialysierflüssigkeit entspricht der Konzentration des Blutes eines Gesunden. Während der Behandlung werden das Blut des Patienten und die Dialysierflüssigkeit an beiden Seiten der Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüberliegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Plasmapherese ist ein Verfahren, wonach das Patientenblut in das Blutplasma und seine korpuskularen Bestandteile (Zellen) aufgetrennt wird. Das abgetrennte Blutplasma wird gereinigt oder durch eine Substitutionslösung ersetzt und dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Die Verfahren zur Dialyse werden in der Regel mit Hilfe von automatischen Dialysegeräten, wie sie beispielsweise von der Anmelderin unter der Bezeichnung 5008 oder sleep.safe vertrieben werden, durchgeführt.

Diese Dialysegeräte sind komplexe medizinische Geräte mit umfangreichen Funktionen. Die Bedienung solcher Geräte geschieht häufig über Touchscreendisplays, die als kombinierte Ein- und Ausgabevorrichtungen zu verstehen sind.

Für die Vorbereitung und die Behandlung selbst sind umfangreiche Bedienschritte und Bedieneingaben in das Dialysegerät notwendig, die in der Regel von geschultem medizinischem Personal durchgeführt werden.

So muss beispielsweise im Falle einer Hämodialysebehandlung das Hämodialysegerät mit für die Behandlung und den Patienten spezifischen Einwegteilen (Disposables) ausgerüstet werden. Hierzu gehören beispielsweise der Blutschlauchsatz und der Dialysefilter.

Weiterhin sind vor oder während der Behandlung diverse Einstellungen am Gerät vorzunehmen. Beispielsweise müssen die typischen Werte für die Flussrate der Blutpumpe oder der Dialysatfluss eingestellt werden. Weiterhin relevant sind die Dialysezeit, die Bluttemperatur im extrakorporalen Blutkreislauf, die Gesamtmenge an Wasser, das dem Patientenblut entnommen werden soll, Flussraten für die Substituatslösung, die gegebenenfalls dem Patientenblut beigemengt wird, oder auch Konzentrationsverhältnisse für die Verdünnung bestimmter dialyse-spezifischer Konzentrate wie Bicarbonat oder Natrium.

Weiterhin kann es während der Behandlung zu bestimmten Situationen kommen, in denen das Dialysegerät Meldungen auf einer Ausgabevorrichtung ausgibt, die zu einer Bedienereingabe, beispielsweise eine Bestätigung bzw. Quittierung oder die Eingabe von Werten durch einen Bediener, auffordern. Eine solche Meldung kann beispielsweise eine Alarmmeldung sein, die beispielsweise über ein Display angezeigt wird. Die Aufmerksamkeit des Bedieners kann aber auch durch eine optische Signalvorrichtung, beispielsweise ein Lampe oder einen Alarmton, erregt werden.

Wesentlich ist, dass zur Abarbeitung solcher Meldungen eine Bedienereingabe in das Dialysegerät unternommen werden muss.

Solche Bedienereingaben werden in der Regel an einem Touchscreendisplay vorgenommen.

Die Behandlung selbst wird oftmals in Dialysezentren vorgenommen, in denen eine Vielzahl von Dialysegeräten für die gleichzeitige Behandlung einer Vielzahl an Patienten vorgehalten werden. Das medizinische Personal bereitet im Rahmen seiner Tätigkeit eine Vielzahl von Behandlungen vor. Oftmals sind hierzu wiederholt für verschiedene Patienten die gleichen Einstellungen am Dialysegerät für die Behandlung vorzunehmen.

Um dem medizinischen Personal hierbei eine Arbeitserleichterung anzubieten, kann es vorgesehen sein, dass das Dialysegerät während der Vorbereitung der Behandlung selbstständig Standardwerte für verschiedene Einstellungen vorschlägt, in dem es diese Werte beispielsweise auf einem Touchscreendisplay anzeigt, die das medizinische Personal durch eine Bedienereingabe, beispielsweise das Betätigen einer OK-Taste auf einem Touchscreendisplay, nur noch bestätigen muss.

Auch während der Behandlung kann es zu Situationen kommen, in denen das Dialysegerät zu einer Bedienereingabe auffordert, beispielsweise wenn eine Überwachungsvorrichtung, die die Behandlung überwacht, einen Alarmzustand detektiert.

Beispielsweise kann während der Dialysebehandlung der arterielle Zulauf-Blutdruck im extrakorporalen Blutkreislauf überwacht werden. Dieser Blutdruck wird auf der Saugseite der Blutpumpe gemessen und hat gegenüber dem Außendruck negative Werte. Ein zu niedriger arterieller Blutdruck lässt gegebenenfalls auf eine zu hohe Blutflussrate schließen. Dementsprechend kann ein Dialysegerät, wenn es einen arteriellen Blutdruck detektiert, der niedriger ist, als ein zuvor festgelegter Grenzwert, eine Alarmmeldung ausgeben.

Gleichzeitig kann das Dialysegerät die Verringerung der Blutflussrate um einen bestimmten Betrag vorschlagen, in dem es eine dementsprechende Ausgabe auf einem Display veranlasst. Dem Bediener können dann über entsprechende Auswahltasten, beispielsweise als Berührflächen auf einem Touchscreendisplay ausgeführt, mehrere Möglichkeiten zum Reagieren auf die Alarmmeldung zur Verfügung gestellt werden. Beispielsweise kann dem von dem Dialysegerät unterbreiteten Vorschlag durch eine Bestätigungs- oder OK-Taste zugestimmt werden, oder durch eine Nein- oder Abbruch-Taste dem Vorschlag widersprochen werden.

Es besteht die Gefahr, dass der Bediener solche Aufforderungen zur bewussten Bestätigung eines von dem Dialysegerät unterbreiteten Vorschlags als störende Unterbrechung eines Handlungsablaufes empfindet und ohne Abwägung, eventuell auch ohne Erfassung der möglichen Folgen des Vorschlages, den Vorschlag vorschnell bestätigt. Damit wird die Absicherung einer automatisch von dem medizinischen Gerät vorgeschlagenen Änderung der Einstellung des medizinischen Geräts behindert.

Dies kann bei medizinischen Geräten, insbesondere bei Dialysegeräten zu einer Gefährdung des Patienten führen, oder zu nicht optimalen Einstellungen, welche die Behandlungsqualität beeinträchtigen können.

Gerade im Alltag in Dialysestationen herrscht durch die Vielzahl von Behandlungen manchmal ein gewisser Zeitdruck, der dazu führen kann, dass von dem Dialysegerät vorgeschlagene Einstellungen vorschnell und unüberlegt akzeptiert und übernommen werden. Dieses Problem stellt sich nicht alleine bei Dialysegeräten, sondern generell bei medizinischen Geräten, die über eine Ausgabevorrichtung, beispielsweise ein Display oder ein Touchscreendisplay, Vorschläge für Einstellungen machen, die vom Bediener nur durch kurzes Bestätigen, beispielsweise durch das Betätigen einer OK-Taste auf einem Touchscreendisplay, angewählt werden können.

Aus dem Stand der Technik sind folgende Dokumente bekannt:
WO 2006/131774 A1 befasst sich mit der Benutzerschnittstelle eines medizinischen Geräts und offenbart Verfahren und Vorrichtungen, das medizinische Gerät mit der Benutzerschnittstelle einzustellen.

EP 0 862 159 A offenbart die Verknüpfung von Daten zu medizinischen Bildern durch Spracheingabe und Spracherkennung.

US 2002/173721 A1 befasst sich mit der Benutzerschnittstelle eines Ultraschallgeräts, die Spracheingaben und handschriftliche Eingaben erlaubt.

US 2008/294458 A1 befasst sich mit der Steuerung von medizinischen Geräten während eines Eingriffs.

US 7 499 862 B1 befasst sich mit einem System zur Erfassung medizinischer Daten basierend auf handschriftlichen und gesprochenen Eingaben.

### Detaillierte Beschreibung der Erfindung

Der Erfindung liegt die Aufgabe zu Grunde, eine Vorrichtung und ein Verfahren zu schaffen, dass einerseits die Arbeit des betreuenden medizinischen Personals bei der Bedienung von medizinischen Geräten unterstützt, andererseits aber sicherstellt, dass bei der Bedienung von medizinischen Geräten, nicht leicht-fertig oder versehentlich für die konkrete Behandlungssituation unpassende Einstellungen vorgenommen werden.

Die Lösung dieser Aufgaben erfolgt erfindungsgemäß durch ein Verfahren mit den Merkmalen des Anspruchs 1 und durch eine Vorrichtung mit den Merkmalen des Anspruchs 5.

Vorteilhafte Ausführungsformen sind Gegenstand der Unteransprüche.

Die Erfindung beruht demnach auf einem Verfahren zur Benutzereingabe in ein medizinisches Gerät mit den Schritten Ausgabe eines ersten das medizinische Gerät kennzeichnenden Parameterwertes, der ein Standardwert ist, handschriftliche Eingabe eines zweiten das Gerät kennzeichnenden Parameterwerts, wonach die Eingabe über eine berührungsempfindliche Fläche erfolgt, und der zweite das Gerät kennzeichnende Parameterwert durch Analysieren der handschriftlichen Eingabe mittels einer im medizinischen Gerät vorgehaltenen Handschrifterkennungssoftware und Übersetzen der Eingabe in einen Zahlenwert durch die Handschrifterkennungssoftware gewonnen wird, Überprüfen des zweiten Parameterwerts, wobei die Überprüfung umfasst, Vergleichen des zweiten das medizinische Gerät kennzeichnenden Parameterwerts mit dem ersten das medizinische Gerät kennzeichnenden Parameterwert, Veranlassen, dass die Steuervorrichtung den ausgegebenen Standardwert zur Parametrisierung eines Steuerprogramms zur Steuerung des medizinischen Geräts übernimmt, wenn der eingegebene zweite das Gerät kennzeichnende Parameterwert mit dem ersten das medizinische Gerät kennzeichnenden Parameterwert übereinstimmt.

Weiterhin beruht die Erfindung auf einer Vorrichtung bestehend aus einem medizinischen Gerät mit einer Eingabevorrichtung, einer Ausgabevorrichtung, einer Handschrifterkennungssoftware und einer Steuervorrichtung, die dazu eingerichtet ist, eine Ausgabe eines ersten das Gerät kennzeichnenden Parameterwerts, der ein Standardwert ist, auf der Ausgabevorrichtung zu veranlassen und einen handschriftlich über die Eingabevorrichtung, die eine berührungsempfindliche Fläche aufweist, eingegebenen zweiten das Gerät kennzeichnenden Parameterwert zu überprüfen, wobei der zweite das Gerät kennzeichnende Parameterwert durch Analysieren der handschriftlichen Eingabe mittels der Handschrifterkennungssoftware und Übersetzen der Eingabe in einen Zahlenwert durch die Handschrifterkennungssoftware gewonnen wird, und wobei die Überprüfung das Vergleichen des ersten das medizinische Gerät kennzeichnenden Parameterwerts mit dem zweiten das medizinische Gerät kennzeichnenden Parameterwert umfasst, und den ersten das Gerät kennzeichnenden Parameterwert, der ein Standardwert ist, in die Steuerung des medizinisches Gerät zur Parametrisierung eines Steuerprogramms zur Steuerung des medizinischen Geräts zu übernehmen, wenn der eingegebene zweite das Gerät kennzeichnende Parameterwert mit dem ersten das medizinische Gerät kennzeichnenden Parameterwert übereinstimmt.

Unter einem medizinischen Gerät sind insbesondere Dialysegeräte zu verstehen. Im Folgenden soll die Erfindung am Beispiel eines zur Hämodialyse eingerichteten Geräts erläutert werden. Dem Fachmann ist klar, dass die Erfindung bei jeglichen medizinischen Geräten anwendbar ist, bei denen sich das Problem stellt, dass vom Gerät gemachte Vorschläge für Einstellungen des jeweiligen Gerätes, die für die konkrete Behandlungssituation unpassende sind, leichtfertig oder versehentlich durch den Bediener übernommen werden.

Solche Einstellungen sind Parameterwerte bestimmter Parameter, die das jeweilige medizinische Gerät kennzeichnen. Die entsprechenden Parameterwerte umfassen beispielsweise Pumpeneinstellungen, wie Blutflussrate, Dialysatflussrate oder Substituatflussrate, aber auch Werte für die Temperatur des extrakorporalen Blutkreislaufes, das Gesamtvolumen an Wasser, das dem Patientenblut während der Behandlung entzogen werden soll, oder die Behandlungsdauer.

Für andere medizinische Geräte können diese Parameterwerte jeweils für andere das jeweilige Gerät spezifische bzw. kennzeichnende Parameter gelten. Bei einem Infusionsgerät sind beispielsweise Infusionsrate und Gesamtvolumen an zu infudierender Flüssigkeit denkbar.

Die Ausgabevorrichtung umfasst oftmals ein Display, welches auch als Touchscreendisplay ausgeführt sein kann. Ein solches Touchscreendisplay kombiniert in einer gemeinsamen Oberfläche eine Ein- und eine Ausgabevorrichtung, in dem es eine berührungsempfindliche Oberfläche bereitstellt, mit der Bedienereingaben detektierbar sind.

Vorstellbar ist aber auch die räumliche Trennung von Ein- und Ausgabevorrichtung, beispielsweise durch ein konventionelles Display, beispielsweise als CRT-Monitor, LCD, Plasma- oder OLED-Display ausgeführt, als Ausgabevorrichtung und einem davon räumlich getrennten Touchpad, das eine berührempfindliche Oberfläche bereitstellt, mit der Bedienereingaben detektierbar sind, als Eingabevorrichtung.

Weiterhin vorstellbar ist, dass die Ausgabevorrichtung einen Lautsprecher umfasst, mit dem von dem medizinischen Gerät vorgeschlagene Parameter und Parameterwerte akustisch als Sprache ausgebbar sind.

Eine solche akustische Ausgabevorrichtung kann zusätzlich zu einer bestehenden optischen Ausgabevorrichtung vorhanden sein.

Zur Eingabe von Bedienerinformationen kann ein Mikrofon vorgesehen sein. Vom Bediener gesprochene Parameterwerte werden hierbei durch eine entsprechend programmierte Steuervorrichtung mit vorgehaltener Spracherkennungssoftware analysiert und weiter verarbeitet.

Medizinische Geräte verfügen oftmals über mindestens ein Steuergerät. Ein solches Steuergerät steuert die Komponenten des medizinischen Geräts unter Anderem anhand derer ihm bekannt gemachten das medizinische Gerät kennzeichnenden Parameterwerte. Solche Steuergeräte sind häufig mit programmierbaren Mikroprozessoren oder Mikrocontrollern ausgerüstet, wobei die sie steuernde Programme in dafür vorgesehenen Programmdatenspeichern abgelegt sind. Hierbei sind verschiedene, die Einstellungen des Geräts betreffende Programme durch die Bedienereingaben parametrisierbar. Diese Bedienereingaben umfassen demnach die das medizinische Gerät kennzeichnenden Parameterwerte.

Oftmals sind auch mehrere Steuergeräte in einem medizinischen Gerät vorgehalten. Im Sinne der Erfindung wird unter Steuergerät auch eine Vielzahl von Steuergeräten verstanden, die durch die Bedienereingaben parametrisierbar sind.

Das Steuergerät hält im Sinne der Erfindung für verschiedene Einstellungen Standardwerte vor, oder bestimmt sie dynamisch, die es im Laufe der Vorbereitung oder während einer medizinischen Behandlung auf der Ausgabevorrichtung vorschlägt. Dies kann optisch durch die Anzeige auf einem Display und/oder auch akustisch durch sprachliche Ausgabe über einen Lautsprecher erfolgen.

Dem Bediener wird so ein Standardwert, der sich für eine Vielzahl von Behandlungssituationen bewährt hat, zur Anzeige gebracht. Dieser Standardwert ist der erste das Gerät kennzeichnende Parameterwert.

Um diesen vorgeschlagenen Parameterwert zur Übernahme in die Steuervorrichtung frei zu geben, wo er ein entsprechendes Programm parametrisiert, oder einen davon unterschiedlichen Wert einzugeben, muss der Bediener erfindungsgemäß den Zahlenwert des ersten Parameters in die Eingabevorrichtung eingeben.

Dies geschieht handschriftlich, in dem der Bediener auf eine berührungsempfindliche Fläche, oftmals als Touchscreendisplay ausgeführt, schreibt. Dies geschieht manuell, beispielsweise mit einem Finger oder einem Eingabestift.

Diese Bedienereingabe wird durch eine im medizinischen Gerät vorgehaltene Handschrifterkennungssoftware, die dem Fachmann bekannt ist, analysiert und in einen Zahlenwert übersetzt. Dieser aus der Bedienereingabe hervorgegangene Zahlenwert ist der zweite das Gerät kennzeichnende Parameterwert.

In einer nicht beanspruchten Ausführungsform kann eine solche Bedienereingabe auch mündlich erfolgen, in dem der Bediener die Eingabe über ein Mikrofon als gesprochene Wörter vornimmt. Eine entsprechend vorgehaltene und dem Fachmann bekannte Spracherkennungssoftware übersetzt diese Eingabe analog der Handschrifterkennungssoftware in den zweiten das Gerät kennzeichnenden Parameterwert.

Die Eingabe des zweiten das Gerät kennzeichnenden Parameterwerts erfolgt also handschriftlich.

Die Steuervorrichtung vergleicht den ersten und den zweiten das Gerät kennzeichnenden Parameterwert und veranlasst für den Fall, dass der erste und der zweite das Gerät kennzeichnende Parameterwert übereinstimmen, dass der erste Parameterwert von einer das Gerät steuernden Steuervorrichtung zur Parametrisierung des entsprechenden Steuerprogramms übernommen wird, oder übernommen werden kann.

Die Übernahme kann also ohne weiteres Zutun des Bedieners erfolgen und sofort Wirkung entfalten, oder sie kann durch eine weitere Bedienereingabe, beispielsweise durch Betätigen einer entsprechenden Taste (beispielsweise als Bedienfläche des Touchscreens ausgeführt) erfolgen. Dies gibt dem Bediener ein weiteres Mal die Gelegenheit, den gerade vorgeschlagenen Parameterwert zu überdenken.

Die Steuervorrichtung kann auch überprüfen, ob der zweite Parameterwert innerhalb bestimmter Grenzwerte liegt. Liegt der zweite Parameterwert außerhalb dieser Grenzwerte, kann die Steuervorrichtung veranlassen, dass weder der erste noch der zweite Parameterwert zur Parametrisierung des entsprechenden Steuer-programms übernommen wird, oder übernommen werden kann.

Im Sinne der Erfindung können die vergleichende bzw. überprüfende und die übernehmende Steuervorrichtung dieselbe Steuervorrichtung sein, es können aber auch unterschiedliche zum medizinischen Gerät gehörende Steuervorrichtungen sein, beispielsweise für den Fall, dass eine Vielzahl von unterschiedlichen Steuervorrichtungen in dem medizinischen Gerät vorgehalten werden.

Die Erfindung beruht also auf der Idee, den Bediener eines medizinischen Geräts dazu zu veranlassen, über den vom Gerät vorgeschlagenen Parameterwert nachzudenken, in dem dieser oder ein anderer Parameterwert des entsprechenden Parameters handschriftlich vom Bediener in das Gerät eingegeben werden, anstatt wie bisher durch einen simple und leichtfertig ausführbare Bestätigung, beispielsweise durch Druck auf eine entsprechende Bedienfläche, auszuführen.

### Kurze Beschreibung der Figuren

Weitere Einzelheiten und Vorteile der Erfindung werden anhand von in den Zeichnungen dargestellten Ausführungsbeispielen näher beschrieben. Es zeigen:
Die Figur 1 ein erfindungsgemäßes medizinisches Gerät, beispielhaft als Dialysegerät ausgeführt;
die Figur 2 den Bildschirminhalt eines medizinischen Geräts nach dem Stand der Technik;
die Figur 3 eine Ausführungsform des Bildschirminhalts eines erfindungsgemäßen medizinischen Geräts;
die Figur 4 zwei weitere Ausführungsformen des Bildschirminhalts eines medizinischen Geräts und
die Figur 5 zwei weitere Ausführungsformen des Bildschirminhalts eines medizinischen Geräts.

### Ausführliche Beschreibung der Figuren

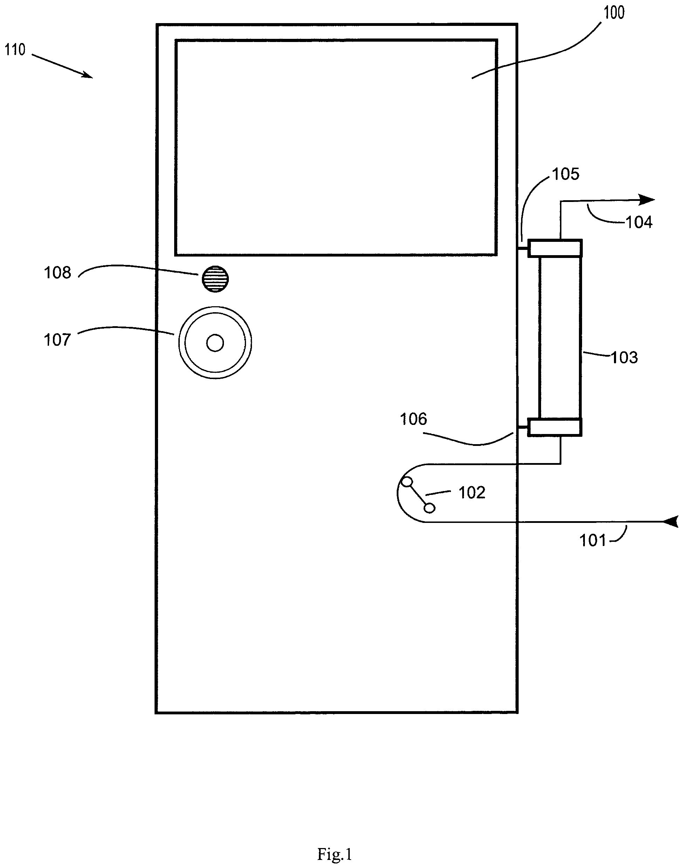

In Figur 1 ist eine Ausführungsform eines erfindungsgemäßen medizinischen Gerätes 110 als Hämodialysegerät mit einem Touchscreendisplay 100 schematisch dargestellt. Die Dialysemaschine 110 zeigt andeutungsweise Teile eines extrakorporalen Blutkreislaufs mit einer arteriellen Blutleitung 101, die Blut eines Patienten (nicht dargestellt) ableitet. Die Blutpumpe 102 fördert das Blut durch einen Dialysefilter 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das Blut des Patienten kann so entwässert werden. Das Dialysat wird in dem Hämodialysegerät 110 hergestellt und nach Gebrauch verworfen. Das Hämodialysegerät 110 ist weiterhin mit einem Lautsprecher 107 und einem Mikrofon 108 als zusätzliche Aus- bzw. Eingabevorrichtung ausgerüstet. Dem Fachmann ist klar, dass ein Lautsprecher grundsätzlich auch als Mikrofon verwendbar ist, da sie nach dem gleichen technischen Prinzip arbeiten können.

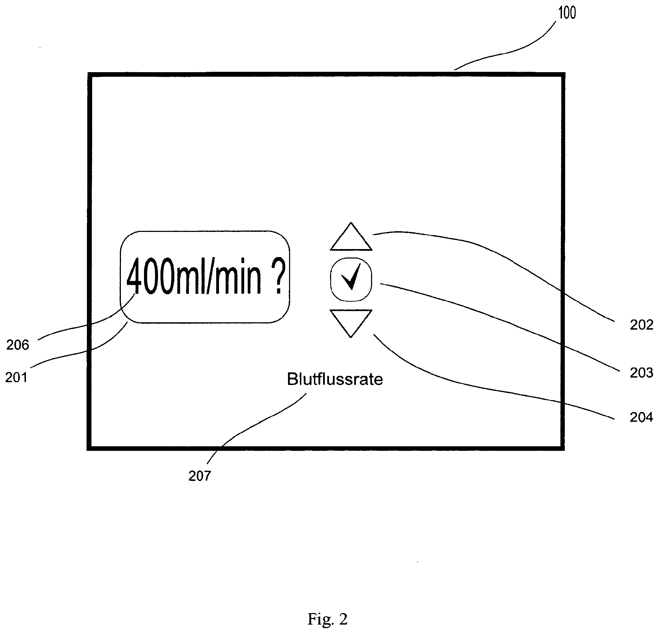

Die Figur 2 zeigt einen für Dialysegerät nach dem Stand der Technik typischen Bildschirminhalt des Touchscreendisplays 100. Im Feld 201 erscheint ein erster das Gerät kennzeichnende Parameterwert 206, hier als Zahlenwert "400" mit angehängter Einheit "ml/min" dargestellt. Das ebenfalls in dem Feld 201 dargestellte Fragezeichen kennzeichnet den Parameter als noch nicht übernommenen Vorschlag. Der zugehörige Parameter 207 (Blutflussrate) wird ebenfalls angezeigt.

Rechts der umrandeten Fläche 201 erscheinen drei zur Bedienereingabe vorgehaltene Flächen. Zur Bestätigung des vorgeschlagenen Parameterwerts 206 muss der Bediener auf die Bedienfläche 203, die mit einem umrandeten Häkchen gekennzeichnet ist, betätigen. Zum Abändern des vorgeschlagenen Parameterwerts 206 dienen die Dreiecksflächen 202 und 204, mit denen der Parameterwert 206 erhöht oder verringert werden kann.

Dieses in Figur 2 dargestellte Bedienkonzept birgt die Gefahr, dass der Bediener zu leichtfertig einen vorgeschlagenen Standardwert übernimmt, da zu dieser Übernahme nur wenige Bedienschritte, im vorliegenden Bespiele nämlich nur einer, notwendig sind.

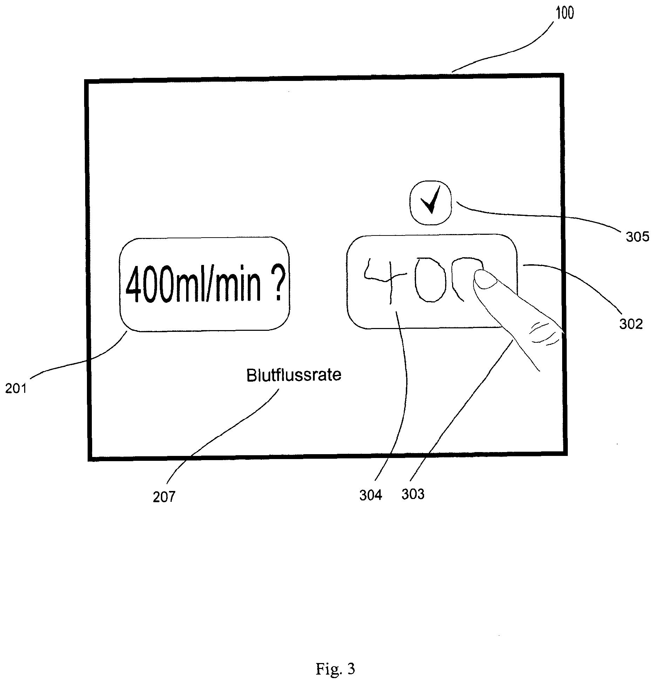

Die Figur 3 zeigt hingegen eine Ausführungsform des Bildschirminhalts eines erfindungsgemäßen medizinischen Geräts für den gleichen Schritt in der Vorbereitung eines medizinischen Geräts, wie er in Figur 2 nach dem Stand der Technik vollführt wird.

Auch hier wird ein Standardwert 206 von der Steuervorrichtung des medizinischen Geräts vorgeschlagen. Im Unterschied zu Figur 1 kann der Bediener diesen Wert aber nicht einfach bestätigen, sondern gibt in einem dafür hier exemplarisch vorgehaltenem Feld 302 per Handschrift einen eigenen zweiten Parameterwert 304 ein.

Dies geschieht durch das Schreiben mit einem Finger 303 auf einer berührungsempfindlichen Fläche, die in Figur 2 als Touchscreendisplay ausgeführt ist.
Die Berührungen des Fingers innerhalb des Feldes 302 werden von einer Steuervorrichtung registriert und als Grafik, die den Parameterwert 304 darstellt, angezeigt.

Hat der Bediener die Eingabe des zweiten Parameterwertes 304 beendet, kann eine Bedienfläche 305 vorgehalten sein, die, wenn sie betätigt wird, einer Steuereinheit meldet, dass die Bedienereingabe vollständig ist. Ein vorgehaltenes Programm zur Erkennung von Handschrift versucht, die Eingabe danach in einen maschinenlesbaren Zahlenwert zu übersetzen.

Stimmt der Zahlenwert, den das Programm zur Erkennung von Handschrift einer Steuereinheit übermittelt hat mit dem ersten Parameterwert 206 überein, veranlasst die Steuereinheit, dass der erste Parameterwert zur Weiterverarbeitung in einer das Gerät steuernde Steuervorrichtung übernommen wird.

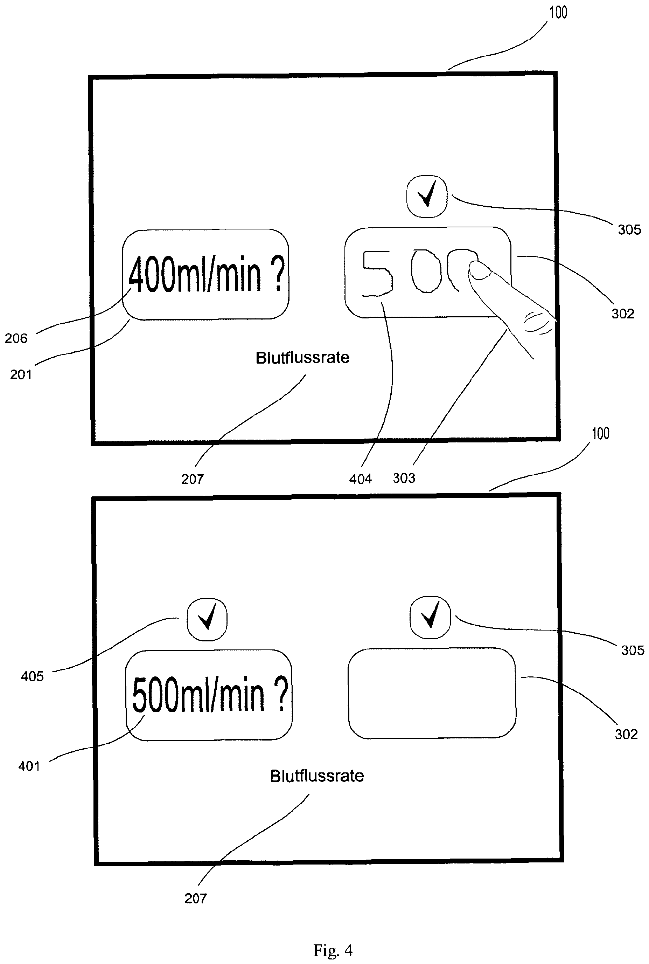

Dies kann ohne Zutun des Bedieners geschehen, oder mit dessen Zutun, in dem er beispielsweise eine zweite Bestätigungsbedienfläche 405 (Figur 4) betätigt.

Es kann vorgesehen sein, dass nach der Überprüfung, ob der Zahlenwert, den das Programm zur Erkennung von Handschrift einer Steuereinheit übermittelt hat, mit dem ersten Parameterwert 206 übereinstimmt, die Ausgabe zumindest eines akustischen, haptischen oder optischen Signals veranlasst wird, dessen wahrnehmbare Eigenschaft vom dem Resultat der Überprüfung abhängt. So kann beispielsweise die Übereinstimmung des ersten und zweiten Parameterwerts durch einen akustischen Ton angezeigt werden, durch eine charakteristische Vibration eines dazu eingerichteten Touchscreendisplays oder durch die Veränderung der Farbe des Feldes 201. Analog kann, wenn erster und zweiter Parameterwert nicht übereinstimmen, ein anderer Ton ausgegeben werden, eine andere Vibration eines dazu eingerichteten Touchscreendisplays veranlasst werden, oder das Feldes 201 in einer andere Farbe als im ersten Fall dargestellt werden. Im Sinne der Erfindung ist die Ausgabe eines Wertes, oder einer Warnung, oder eines Symbols auf dem Display 100, welche von dem Resultat der Überprüfung abhängt, ebenfalls ein optisches Signal. Die Ausgabe eines akustischen, haptischen oder optischen Signals kann in einer nicht beanspruchten Ausführungsform in analoger Weise auch von der Überprüfung einer durch den Bediener eingegebenen Anweisung erfolgen, indem beispielsweise überprüft wird, ob die eingegebene Anweisung ausführbar ist.

In gleicher Weise können in einer nicht beanspruchten Ausführungsform akustische, haptische und/oder optische Signale veranlasst werden, deren wahrnehmbare Eigenschaften vom dem Resultat einer Überprüfung, ob der eingegebene zweite Parameterwert innerhalb von Grenzwerten liegt, abhängt.

In Figur 4 ist die Situation gezeigt, wenn der eingegebene zweite Parameterwert 404 vom Vorschlag 206 des Steuergeräts abweicht. Im oberen Bild der Figur 4 gibt der Bediener statt des vorgeschlagenen Parameterwerts 206 ("400") den davon abweichenden Wert 404 ("500") ein.

Wie bei Figur 3 schon beschrieben, übersetzt ein Programm zur Erkennung von Handschrift die Bedienereingabe in einen maschinenlesbaren Zahlenwert 401 und bringt ihn im Feld 201 zur Anzeige. Gleichzeitig kann das Feld zur Eingabe des Bedieners 302 von der Anzeige der Handschrift, die den Parameterwert 404 darstellt, bereinigt werden, um eine erneute Eingabe im Feld 302 zu ermöglichen.

Der Bediener hat nun die Möglichkeit, den neuen aus seiner ursprünglichen Eingabe erkannten Wert 401 zu bestätigen, in dem er das entsprechende Feld 405 betätigt, oder aber seine Eingabe zu wiederholen und nach wiederholter Eingabe das Feld 305 in schon bei Figur 3 beschriebener Weise zu betätigen.

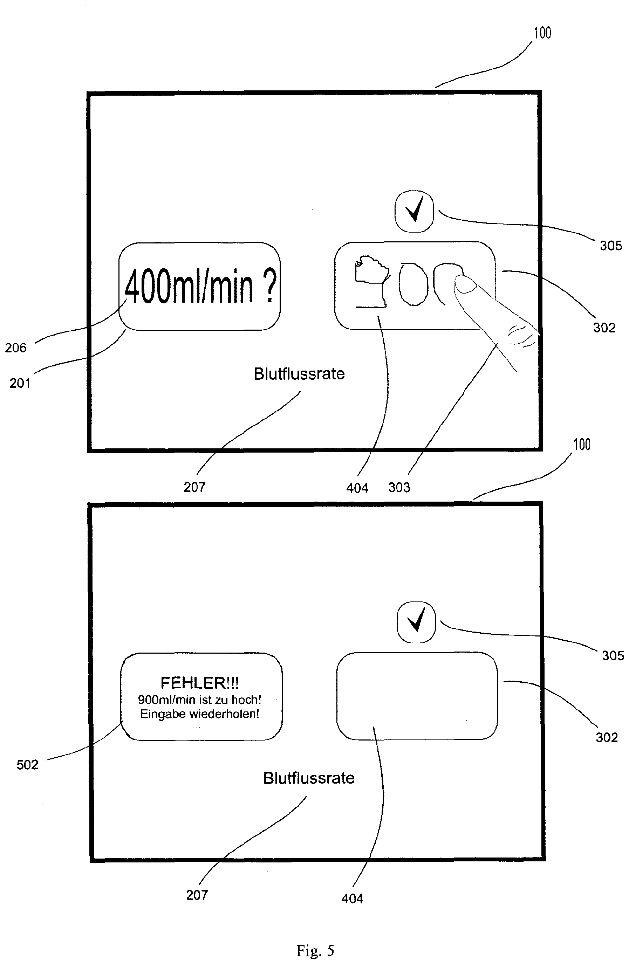

Ein über das Feld 405 bestätigter vom Bediener eingegebener Zahlenwert kann dann als zweiter Parameterwert von einer das Gerät steuernde Steuervorrichtung übernommen werden. Die Figur 5 zeigt zwei weitere Ausführungsformen des Bildschirminhalts eines medizinischen Geräts in einer nicht beanspruchten Ausführungsform, für die Situation, wenn der vom Bediener eingegebene Zahlenwert außerhalb eines zuvor der Steuervorrichtung bekannt gemachten Bereichs liegt.

In der Figur 5 im oberen Bild schlägt die Steuervorrichtung als ersten Parameterwert 206 den Zahlenwert "400" vor, der Bediener gibt aber davon abweichend einen zweiten Parameterwert mit dem Zahlenwert "900" in das Feld 302 ein.

Die Steuervorrichtung kann so eingerichtet sein, dass jegliche Eingabe des Bedieners auf Plausibilität und Einhaltung von zuvor programmierten Grenzwerten überprüft wird. So kann es sich beispielsweise bei dem Parameter 207, dessen Wert in der Figur 5 eingegeben wird, um die Blutflussrate im extrakorporalen Blutkreislauf handeln, wie in den Figuren 2 bis 5 dargestellt.

Das Steuergerät kann den eingegebenen Parameterwert mit für die Behandlung und für den jeweiligen Patienten zuvor programmierten Grenzwerten vergleichen und eine Warnung 502 (in Figur 5 im unteren Bild) ausgeben, wenn der eingegebene Parameterwert außerhalb der Grenzwerte liegt.

So ist beispielsweise für eine Hämodialysebehandlung Erwachsener eine Blutflussrate bis maximal 700ml/min typisch. Für Kinder gelten wiederum andere Grenzwerte. Die Grenzwerte können individuell ablegt werden und stehen der Steuervorrichtung bei jeder Behandlung zur Verfügung.

Im Falle einer solchen Grenzwertverletzung kann wie in Figur 5 unten dargestellt die Eingabefläche 302 gelöscht werden und zur erneuten Eingabe aufgefordert werden.

Weiterhin kann überprüft werden, ob ein eingegebener zweiter Parameterwert zur Nichteinhaltung therapeutischer Ziele führt, wenn dieser Wirkung in der Maschine erlangen würde. Oftmals verschreibt der behandelnde Arzt auf Basis der Therapieparameter eine bestimmte Zeitspanne, während derer sich der Patient der Dialyse unterziehen soll, um eine bestimmtes Therapieziel zu erreichen.

Während der Behandlung können aber bestimmte Situationen aufkommen, beispielsweise Kreislaufschwankungen des Patienten, die ein Abweichen von der ursprünglichen ärztlichen Verschreibung notwendig macht. Beispielsweise kann der Dialysatfluss verringert werden, um eine zu schnelle Entfernung bestimmter Stoffe aus dem Blut zu verhindern.

Ein verringerter Dialysatfluss hat zur Folge, dass das ursprüngliche Therapieziel nicht erreicht wird, wenn die Dialysezeit nicht verlängert wird. Eine solche Abweichung kann als Warnung über eine Anzeigevorrichtung an den Bediener ausgegeben werden. Analog zur Meldung 502 der Figur 5 kann darauf hingewiesen werden, dass der vom Bediener eingegebene Parameterwert zur Nichteinhaltung der ärztlichen Verschreibung führt, wenn er in dem medizinischen Gerät Wirkung erlangt. Eine derartige an den Bediener des medizinischen Geräts, das hier beispielhaft als Dialysemaschine ausgeführt ist, gerichtete Meldung kann beispielsweise wie folgt formuliert sein:
"Der von Ihnen eingegebene neue Dialysatfluss von 300ml/min hat bei unveränderter Dialysezeit zur Folge, dass das erwünschte Therapieziel nicht erreicht werden wird. Soll die Dialysezeit um 1 Stunde verlängert werden, um das ursprüngliche Therapieziel zu erreichen? Bitte "Ja" "Nein" oder "Abbrechen" eingeben."

Der Bediener gibt dann statt einer Zahl ein Wort als Anweisung ein, um ausgehend von obiger Meldung die Dialysezeit anzupassen (Eingabe "Ja"), die Dialysezeit unverändert zu lassen (Eingabe "Nein") oder den Vorgang abzubrechen (Eingabe "Abbrechen"). Die Steuervorrichtung des medizinischen Geräts überprüft anschließend in einer nicht beanspruchten Ausführungsform die Eingabe des Bedieners daraufhin, ob sie einer Anweisung entspricht, die umsetzbar ist und übernimmt abhängig von dieser Überprüfung die entsprechende Anweisung.

Es kann während der Behandlung darüber hinaus auch beispielsweise zu Situationen kommen, in denen der Bediener die Behandlung zunächst abbrechen möchte. Das medizinische Gerät kann in einem solchen Fall bevor es die Behandlung tatsächlich abbricht den Bediener über die Folgend eines Behandlungsabbruches informieren, in dem es eine entsprechende Meldung auf einer Anzeigevorrichtung ausgibt und auf eine Bestätigung durch eine Bedienereingabe wartet.

Die Folge eines Behandlungsabbruches ist häufig, dass eine Verschreibung des Arztes nicht eingehalten wird.

Eine typische Verschreibung für eine Dialysebehandlung ist das zu erreichende Gewicht des Patienten, der aufgrund seiner Niereninsuffizienz mit der Nahrung aufgenommenes Wasser nicht mehr ausreichend ausscheiden kann. Dieses Wasser reichert sich im Blut und im Gewebe des Patienten an. Der Patient hat zu Beginn der Behandlung folglich ein Körpergewicht, das über dem eines Vergleichspatienten mit normaler Nierentätigkeit liegt. Ein Ziel der Dialyse ist es, das überschüssige Wasser aus dem Patientenblut zu entfernen. Die Menge des entfernten Wassers entspricht dem Gewichtsverlust des Patienten während der Dialyse. Deshalb verschreibt der Dialysearzt häufig ein zu erreichendes Endgewicht, auch Trockengewicht oder Sollgewicht genannt.

Bricht der Bediener die Behandlung vorzeitig ab, kann das dazu führen, dass dieses verschriebene Gewicht des Patienten nicht erreicht wird.

Um den Bediener über die Konsequenzen des Abbruchs zu informieren, kann das Dialysegerät eine Meldung ausgeben. Diese Meldung kann Schlüsselwörter enthalten. Diese Schlüsselwörter können beispielsweise "Gewicht" oder "Fortsetzen" sein. Die Schlüsselwörter charakterisieren die Ihrem Wortsinn entsprechenden Konsequenzen. Eine derartige Meldung könnte wie folgt lauten:
"Bei sofortigem Abbruch der Behandlung wird das verschriebene GEWICHT nicht erreicht. Zur Bestätigung bitte GEWICHT eingeben. Soll die Behandlung fortgesetzt werden bitte FORTSETZEN eingeben."

Um den Abbruch zu bestätigen, muss der Bediener in schon beschriebener Weise das Schlüsselwort "GEWICHT" in eine Eingabevorrichtung eingeben, bei Eingabe des Schlüsselwortes "FORTSETZEN" wird die Behandlung fortgesetzt.

Durch die Eingabe des Schlüsselworts wird dem Bediener also erneut Gelegenheit gegeben, die Konsequenzen seiner Handlung zu überdenken. Dies ist insbesondere deswegen der Fall, weil das einzugebende Schlüsselwort die Konsequenz der Eingabe charakterisiert. Das vermindert die Gefahr übereilter und unreflektierter Handlungen. Dies betrifft nicht nur das von einem Bediener initiierte vorzeitige Abbrechen einer Behandlung, sondern jeglichen Bedienereingriff.

Die handschriftliche oder mündliche Eingabe eines Schlüsselwortes ist die Eingabe einer Anweisung.

Bei Eingaben des Bedieners, bei denen das vorgehaltene Programm zur Erkennung von Handschrift keinen Zahlenwert oder keine Anweisung erkennen kann, beispielsweise durch unvollständige oder nicht leserliche handschriftliche Eingabe, kann eine Warnung ausgegeben werden und zur erneuten Eingabe aufgefordert werden.

In einer Ausführungsform der Erfindung erkennt das vorgehaltene Programm zur Erkennung von Handschrift anhand der eingegebenen Handschrift die Person, die die Eingabe vorgenommen hat, bzw. erkennt, dass die Person, die die Eingabe vorgenommen hat, nicht zu den Personen gehört, die Eingaben vornehmen dürfen.

Beispielsweise kann es vorgesehen sein, dass nur bestimmte Personen, wie Ärzte oder betreuendes medizinisches Personal, Eingaben in das medizinische Gerät vornehmen dürfen. Die Charakteristika der Handschrift dieser Personen können abgespeichert sein. Das vorgehaltene Programm zur Erkennung von Handschrift kann auf diese Weise erkennen, welche dazu vorgesehene Person Eingaben vornimmt, oder ob eine nicht dafür vorgesehene Person Eingaben vornimmt.

Derart kann ein Protokoll angelegt und elektronisch abgespeichert werden, welche Person Eingaben in das medizinische Gerät vorgenommen hat, oder es kann verhindert werden, dass nicht dazu berechtigte Personen Eingaben in das medizinische Gerät vornehmen, indem die Übernahme von Eingaben nicht berechtigter Personen verweigert wird.

Die in den Figuren 3 bis 5 beschriebene handschriftliche Bedienereingabe kann in einer nicht beanspruchten Ausführungsform durch eine mündliche Bedienereingabe ergänzt oder ersetzt werden, in dem alternativ oder zusätzlich zur optischen Ausgabe vorgeschlagener Werte diese Werte akustisch über Sprachausgabe durch einen Lautsprecher 107 ausgegeben werden und alternativ oder zusätzlich zu der handschriftlichen Bedienereingabe diese Werte akustisch durch gesprochene Zahlenwerte über ein Mikrofon 108 eingegeben werden.

Die Ausgabe der vorgeschlagenen Parameterwerte bestimmter erster Parameter kann durch die Benennung des zu dem ersten Parameterwert gehörenden Parameters unterstützt werden.

So wird bei dem Parameter Blutflussrate das Wort ""Blutflussrate" über den Lautsprecher ausgegeben und mit einem nachfolgenden Wert, beispielsweise "400" verknüpft.

Die Bedienereingabe kann hiernach dadurch erfolgen, in dem der Bediener den zweiten Parameterwert oder eine Anweisungen ausspricht. Über das vorgehaltene Mikrofon 108 und eine entsprechendes Programm kann aus der gesprochenen Eingabe eine maschinenlesbare Zahl oder eine Anweisung bestimmt werden. Mit einer Zahl als zweiten Parameterwert kann in derselben Weise verfahren werden, wie bei handschriftlicher Eingabe des zweiten Parameterwertes.

Dies betrifft insbesondere auch die Fälle, wo die mündliche Eingabe unverständlich ist, wobei die Steuervorrichtung akustisch dazu auffordern kann, die Eingabe zu wiederholen, oder wo der zweite Parameterwert außerhalb von Grenzwerten ist, wobei die Steuervorrichtung eine Warnung aussprechen kann. Ebenso kann durch die Analyse des gesprochenen Wortes analog zur Handschriftenerkennung auf die sprechende Person geschlossen werden und folglich in wie schon zuvor beschriebener Weise verhindert werden, dass Eingaben von einer nicht dafür vorgesehenen Person getätigt werden.

Darüber hinaus offenbart die Kombination von handschriftlicher und mündlicher Eingabe eine weitere Möglichkeit, die Sicherheit der Eingabe von Bedienern zu erhöhen. In diesem Fall muss der Bediener sowohl eine handschriftliche Eingabe, als auch eine mündliche Eingabe vornehmen.

Handschriftliche und mündliche Eingabe werden hierbei in schon beschriebener Weise jede für sich und zusätzlich auf Konsistenz der beiden Eingaben geprüft. Nur wenn die handschriftliche und die mündliche Eingabe übereinstimmen, kann die Steuervorrichtung die Weitergabe des eingegebenen Parameterwertes erlauben.

Mit Hilfe der Erfindung wird somit einerseits die Arbeit des betreuenden medizinischen Personals bei der Bedienung unterstützt, andererseits aber sicherstellt, dass bei der Bedienung nicht leichtfertig oder versehentlich für die konkrete Behandlungssituation unpassende Einstellungen vorgenommen werden.

Die Ausführungsformen sind als Beispiele zu verstehen, dem Fachmann ist es nahe gelegt, diese Ausführungsformen ohne weiteres erfinderisches Zutun auf nicht dargelegte andere Ausführungsformen anzuwenden.

## Patentansprüche

1. Verfahren zur Übernahme eines vorgeschlagenen Standardwerts in die Steuervorrichtung eines medizinischen Geräts mit den Schritten:
Ausgabe eines ersten das medizinische Gerät kennzeichnenden Parameterwertes, der ein Standardwert ist,
handschriftliche Eingabe eines zweiten das Gerät kennzeichnenden Parameterwerts, wobei die Eingabe über eine berührungsempfindliche Fläche erfolgt, und der zweite das Gerät kennzeichnende Parameterwert durch Analysieren der handschriftlichen Eingabe mittels einer im medizinischen Gerät vorgehaltenen Handschrifterkennungssoftware und Übersetzen der Eingabe in einen Zahlenwert durch die Handschrifterkennungssoftware gewonnen wird, Überprüfen des zweiten Parameterwerts, wobei die Überprüfung umfasst,
Vergleichen des zweiten das medizinische Gerät kennzeichnenden Parameterwerts mit dem ersten das medizinische Gerät kennzeichnenden Parameterwert,
Veranlassen, dass die Steuervorrichtung den ausgegebenen Standardwert zur Parametrisierung eines Steuerprogramms zur Steuerung des medizinischen Geräts übernimmt, wenn der eingegebene zweite das Gerät kennzeichnende Parameterwert mit dem ersten das medizinische Gerät kennzeichnenden Parameterwert übereinstimmt.

2. Verfahren nach Anspruch 1, wonach die Ausgabe auf einem Display oder über einen Lautsprecher erfolgt.

3. Verfahren nach einem der vorhergehenden Ansprüche, wonach nach der Überprüfung zumindest ein akustisches, haptisches oder optisches Signal ausgeben wird, dessen wahrnehmbare Eigenschaft von dem Resultat des Vergleichs abhängt.

4. Verfahren nach einem der vorhergehenden Ansprüche, wonach mittels einer im medizinischen Gerät vorgehaltene Handschrifterkennungssoftware anhand der handschriftlichen Eingabe erkannt wird, ob die handschriftliche Eingabe von einer Person vorgenommen wurde, deren Handschriftcharakteristika abgespeichert sind.

5. Vorrichtung zur Übernahme eines vorgeschlagenen Standardwerts in die Steuervorrichtung eines medizinischen Geräts bestehend aus einem medizinischen Gerät mit einer Eingabevorrichtung, einer Ausgabevorrichtung, einer Handschrifterkennungssoftware und einer Steuervorrichtung, die dazu eingerichtet ist, eine Ausgabe eines ersten das Gerät kennzeichnenden Parameterwerts, der ein Standardwert ist, auf der Ausgabevorrichtung zu veranlassen und einen handschriftlich über die Eingabevorrichtung, die eine berührungsempfindliche Fläche aufweist, eingegebenen zweiten das Gerät kennzeichnenden Parameterwert zu überprüfen, wobei der zweite das Gerät kennzeichnende Parameterwert durch Analysieren der handschriftlichen Eingabe mittels der Handschrifterkennungssoftware und Übersetzen der Eingabe in einen Zahlenwert durch die Handschrifterkennungssoftware gewonnen wird, und wobei die Überprüfung das Vergleichen des ersten das medizinische Gerät kennzeichnenden Parameterwerts mit dem zweiten das medizinische Gerät kennzeichnenden Parameterwert umfasst, und den ersten das Gerät kennzeichnenden Parameterwert, der ein Standardwert ist, in die Steuerung des medizinisches Gerät zur Parametrisierung eines Steuerprogramms zur Steuerung des medizinischen Geräts zu übernehmen, wenn der eingegebene zweite das Gerät kennzeichnende Parameterwert mit dem ersten das medizinische Gerät kennzeichnenden Parameterwert übereinstimmt.

6. Vorrichtung nach Anspruch 5, wonach die Ausgabevorrichtung ein Display oder ein Lautsprecher ist.

7. Vorrichtung nach einem der Ansprüche 5 oder 6, wonach die Steuervorrichtung dazu eingerichtet ist, nach der Überprüfung die Ausgabe eines akustischen, haptischen und/oder optischen Signals zu veranlassen, dessen wahrnehmbare Eigenschaft vom dem Resultat der Überprüfung abhängt.

8. Vorrichtung nach einem der Ansprüche 5 bis 7, wonach die Steuervorrichtung mittels einer im medizinischen Gerät vorgehaltene Handschrifterkennungssoftware dazu eingerichtet ist anhand der handschriftlichen Eingabe zu erkennen, ob die handschriftliche Eingabe von einer Person vorgenommen wurde, deren Handschriftcharakteristika abgespeichert sind.

9. Vorrichtung nach einem der Ansprüche 5 bis 8, wobei das medizinische Gerät ein Blutbehandlungsgerät ist.

10. Vorrichtung nach Anspruch 9, wobei das Blutbehandlungsgerät zur Hämodialyse, Peritonealdialyse oder Plasmapherese eingerichtet ist.

## Claims

1. Method for adopting a proposed standard value in the control apparatus of a medical device, including the steps of:
outputting a first parameter value characterizing the medical device, said first parameter value being a standard value,
handwritten inputting of a second parameter value characterizing the device, wherein the input is implemented by way of a touch-sensitive area and the second parameter value characterizing the device is obtained by analysing the handwritten input by means of handwriting recognition software kept available in the medical device and translating the input into a numerical value by way of the handwriting recognition software,
checking the second parameter value, the check comprising
comparing the second parameter value characterizing the medical device to the first parameter value characterizing the medical device,
prompting the control apparatus to adopt the output standard value for parameterizing a control program for controlling the medical device if the input second parameter value characterizing the device corresponds to the first parameter value characterizing the medical device.

2. Method according to Claim 1, in which the output is implemented on a display or by way of a loudspeaker.

3. Method according to either of the preceding claims, in which at least one acoustic, haptic or optical signal is output following the check, the perceivable property of said signal depending on the result of the comparison.

4. Method according to any one of the preceding claims, in which handwriting recognition software kept available in the medical device uses the handwritten input to identify whether the handwritten input was provided by a person whose handwriting characteristics are stored.

5. Apparatus for adopting a proposed standard value in the control apparatus of a medical device, consisting of a medical device with an input apparatus, an output apparatus, handwriting recognition software and a control apparatus configured to prompt an output of a first parameter value characterizing the device, which parameter value is a standard value, on the output apparatus and to check a second parameter value characterizing the device, which was input in handwritten form by way of the input apparatus with a touch-sensitive area, wherein the second parameter value characterizing the device is obtained by analysing the handwritten input by means of the handwriting recognition software and translating the input into a numerical value by way of the handwriting recognition software and wherein the check comprises comparing the first parameter value characterizing the medical device to the second parameter value characterizing the medical device, and adopting the first parameter value characterizing the device, which parameter value is a standard value, in the control of the medical device for parameterizing a control program for controlling the medical device if the input second parameter value characterizing the device corresponds to the first parameter value characterizing the medical device.

6. Apparatus according to Claim 5, in which the output apparatus is a display or a loudspeaker.

7. Apparatus according to either of Claims 5 and 6, in which the control apparatus is configured to prompt the output of an acoustic, haptic and/or optical signal following the check, the perceivable property of said signal depending on the result of the check.

8. Apparatus according to any one of Claims 5 to 7, in which the control apparatus is configured by means of handwriting recognition software kept available in the medical device to identify, on the basis of the handwritten input, whether the handwritten input was provided by a person whose handwriting characteristics are stored.

9. Apparatus according to any one of Claims 5 to 8, wherein the medical device is a blood treatment device.

10. Apparatus according to Claim 9, wherein the blood treatment device is configured for haemodialysis, peritoneal dialysis or plasmapheresis.

## Revendications

1. Procédé permettant d'adopter une valeur par défaut proposée dans le dispositif de commande d'un appareil médical, comprenant les étapes consistant à :
sortir une première valeur de paramètre caractérisant l'appareil médical qui est une valeur par défaut,
saisir à la main une deuxième valeur de paramètre caractérisant l'appareil, la saisie étant effectuée par l'intermédiaire d'une surface tactile, et la deuxième valeur de paramètre caractérisant l'appareil étant obtenue en analysant la saisie manuscrite au moyen d'un logiciel de reconnaissance d'écriture manuscrite prévu dans l'appareil médical et en traduisant la saisie en une valeur numérique par le logiciel de reconnaissance d'écriture manuscrite,
vérifier la deuxième valeur de paramètre, la vérification comprenant
la comparaison de la deuxième valeur de paramètre caractérisant l'appareil médical à la première valeur de paramètre caractérisant l'appareil médical,
le fait d'amener le dispositif de commande à adopter la valeur par défaut sortie pour paramétrer un programme de commande pour commander l'appareil médical si la deuxième valeur de paramètre saisie caractérisant l'appareil coïncide avec la première valeur de paramètre caractérisant l'appareil médical.

2. Procédé selon la revendication 1, dans lequel la sortie est effectuée sur un affichage ou par un haut-parleur.

3. Procédé selon l'une quelconque des revendications précédentes, dans lequel, après la vérification, au moins un signal acoustique, haptique ou optique est sorti dont la propriété perceptible dépend du résultat de la comparaison.

4. Procédé selon l'une quelconque des revendications précédentes, dans lequel un logiciel de reconnaissance d'écriture manuscrite prévu dans l'appareil médical permet de reconnaître à l'aide de la saisie manuscrite si la saisie manuscrite a été effectuée par une personne dont les caractéristiques d'écriture manuscrite sont enregistrées.

5. Dispositif permettant d'adopter une valeur par défaut proposée dans le dispositif de commande d'un appareil médical, composé d'un appareil médical muni d'un dispositif de saisie, d'un dispositif de sortie, d'un logiciel de reconnaissance d'écriture manuscrite et d'un dispositif de commande, qui est conçu pour provoquer une sortie d'une première valeur de paramètre caractérisant l'appareil, qui est une valeur par défaut, sur le dispositif de sortie, et pour vérifier une deuxième valeur de paramètre caractérisant l'appareil, saisie à la main par l'intermédiaire du dispositif de saisie qui présente une surface tactile, la deuxième valeur de paramètre caractérisant l'appareil étant obtenue en analysant la saisie manuscrite au moyen du logiciel de reconnaissance d'écriture manuscrite et en traduisant la saisie en une valeur numérique par le logiciel de reconnaissance d'écriture manuscrite, et la vérification comprenant la comparaison de la première valeur de paramètre caractérisant l'appareil médical à la deuxième valeur de paramètre caractérisant l'appareil médical, et pour adopter la première valeur de paramètre caractérisant l'appareil, qui est une valeur par défaut, dans l'unité de commande de l'appareil médical pour paramétrer un programme de commande pour commander l'appareil médical si la deuxième valeur de paramètre saisie caractérisant l'appareil coïncide avec la première valeur de paramètre caractérisant l'appareil médical.

6. Dispositif selon la revendication 5, dans lequel le dispositif de sortie est un affichage ou un haut-parleur.

7. Dispositif selon l'une quelconque des revendications 5 ou 6, dans lequel le dispositif de commande est conçu, après la vérification, pour provoquer la sortie d'un signal acoustique, haptique et/ou optique dont la propriété perceptible dépend du résultat de la vérification.

8. Dispositif selon l'une quelconque des revendications 5 à 7, dans lequel le dispositif de commande est conçu au moyen d'un logiciel de reconnaissance d'écriture manuscrite prévu sur l'appareil médical pour reconnaître à l'aide de la saisie manuscrite si la saisie manuscrite a été effectuée par une personne dont les caractéristiques d'écriture manuscrite sont enregistrées.

9. Dispositif selon l'une quelconque des revendications 5 à 8, dans lequel l'appareil médical est un appareil de traitement du sang.

10. Dispositif selon la revendication 9, dans lequel l'appareil de traitement du sang est conçu pour l'hémodialyse, la dialyse péritonéale ou la plasmaphérèse.
